# FASCICULE DE BREVET EUROPEEN

(11) **EP 4 281 744 B1**
(45) Date de publication et mention de la délivrance du brevet: **01.10.2025**
(21) Numéro de dépôt: 22700994.1
(22) Date de dépôt: 21.01.2022
(51) Int. Cl.: G01N 15/06, G01N 15/1434, G01N 15/14, G01N 21/47, G01N 21/53, G01N 33/24, G01N 15/00

(54) **SYSTÈME DE DÉTECTION SUR SITE DE PARTICULES D'AMIANTE DANS DES MATÉRIAUX DE CONSTRUCTION**
SYSTEM ZUR DETEKTION VON ASBESTPARTIKELN IN BAUSTOFFEN VOR ORT
SYSTEM FOR DETECTING ON-SITE ASBESTOS PARTICLES IN BUILDING MATERIALS

(30) Priorité: 22.01.2021 FR 2100625
(43) Date de publication de la demande: 29.11.2023
(73) Titulaire: BRGM, 45060 Orléans Cedex 2 (FR); Centre National de la Recherche Scientifique, 75016 Paris (FR); Université d'Orléans, 45067 Orléans (FR)
(72) Inventeur: DUEE, Cédric, 45240 Marcilly-en-Villette (FR); HAAS, Hubert, 45100 ORLEANS - La Source (FR); RENARD, Jean-Baptiste, 45000 ORLEANS (FR)
(74) Mandataire: LLR
(86) Numéro de dépôt international: PCT/EP2022/051356
(87) Numéro de publication internationale: WO 2022/157317

(56) Documents cités:
- WO-A1-98/20320
- GB-A- 2 403 289
- NL-C2- 1 017 770
- US-A1- 2010 085 569
- US-A1- 2011 310 386

## Description

L'invention concerne la détection d'amiante, plus particulièrement lors des diagnostics amiante règlementaires dans le cadre de la surveillance de bâti ancien, de travaux et interventions liés à des matériaux susceptibles de contenir de l'amiante, de démolition ou en cas de catastrophe naturelle.

L'amiante est une famille de minéraux naturels présents dans les roches et les sols, silicates appartenant à deux groupes :
- les amphiboles (actinolite-amiante, anthophyllite-amiante, trémolite-amiante, amosite, crocidolite),
- les serpentines (chrysotile , antigorite en Nouvelle Calédonie).

Le plus utilisé dans le bâtiment est le chrysotile (pour 90 à 95 %), puis l'amosite dans les navires, et la crocidolite. Le chrysotile et l'amosite représentent 98 % de l'amiante des bâtiments, et avec la crocidolite, ces trois amiantes représentent environ 99% de l'amiante utilisé dans les bâtiments.

En France, l'amiante est présent dans de nombreux bâtiments et équipements construits avant 1997. Lors de l'ouverture d'un chantier ou après les travaux, le maître d'œuvre doit faire établir un diagnostic amiante dans un certain nombre de cas prévus par la loi pour faire respecter les normes sur le chantier. Dans les autres cas, même s'il n'est pas obligatoire, un test peu onéreux serait une véritable innovation et un bénéfice pour l'ensemble de la filière. Ainsi, lors de travaux sur des matériaux susceptibles de contenir de l'amiante (fibrociments, flocages, calorifugeages, faux plafonds, dalles, toitures, chantiers de désamiantages...), un test rapide, portable sur site, capable d'identifier l'émissivité du matériau, constitue un progrès considérable pour établir un diagnostic sur ou à proximité d'un site suspecté de présenter de l'amiante.

On connaît déjà dans l'état la technique un appareil commercialisé pour la détection d'amiante dans les matériaux du bâtiment sur site et en temps réel, basé sur la spectroscopie infrarouge. Cependant il a été montré que cet appareil ne distingue ni la taille nanométrique de l'amiante, ni la morphologie fibreuse, ni les silicates ordinaires présentant les mêmes modes de vibration infrarouge que l'amiante, et a tendance à générer de nombreux faux négatifs.

A l'heure actuelle, lors d'un diagnostic amiante réglementaire (normes NF X 43-050, NF ISO 22262-1 et 2 par exemple), on effectue un prélèvement sur site, puis ce prélèvement est envoyé en laboratoire pour analyse par microscopie (microscopie optique à lumière polarisée - MOLP, microscopie électronique à balayage - MEB, ou microscopie électronique en transmission - MET), ce qui génère un délai d'attente pour l'obtention du résultat pouvant aller jusqu'à 48 heures. Dans ces procédés réglementaires, on n'effectue donc pas de mesure sur site.

L'invention a notamment pour but de fournir un système pour la détection d'amiante sur site, permettant une détection fiable et rapide de l'amiante.

A cet effet l'invention a pour objet un système de détection sur site de particules d'amiante dans des matériaux de construction comprenant :
- un moyen pour libérer les particules d'amiante présentes dans les matériaux,
- une pompe configurée pour prélever des particules solides libérées par ledit moyen,
- un système d'analyse des particules solides comprenant :
   une source de lumière configurée pour générer un champ lumineux initial,
   un moyen principal de détection d'un champ lumineux diffusé par lesdites particules solides, le moyen principal de détection étant orienté dans une direction formant un angle inférieur à 30° par rapport à la direction du champ lumineux initial, appelé premier angle,
   un moyen complémentaire de détection du champ lumineux diffusé par lesdites particules solides, le moyen complémentaire de détection étant orienté dans une direction formant un angle entre 45° et 90° par rapport à la direction du champ lumineux initial, appelé second angle.

On entend par « système de détection sur site » un système transportable par un opérateur, présentant un faible encombrement, c'est-à-dire un volume inférieur à 1 m³, et un poids inférieur à 15 kg. Le système de détection sur site est également rapidement installé par un opérateur, c'est-à-dire en moins de 30 minutes.

Les particules peuvent être des fibres ou des particules issues de fibres. On comprend que les particules solides libérées comprennent les particules d'amiante libérées.

On comprend que le système d'analyse des particules solides est configuré pour détecter un champ lumineux diffusé par des particules solides au moins en deux angles distincts. En fonction des analyses, il peut être pertinent de détecter le champ lumineux diffusé en plus de deux angles.

Ce système présente l'avantage de permettre une détection rapide, fiable et peu onéreuse de l'amiante. Par ailleurs, la détection étant faite sur site, elle permet un tri des déchets sans avoir à transporter des échantillons, ce qui simplifie la logistique.

Les inventeurs ont choisi le deuxième angle car il permet de différencier au mieux des particules de sable des particules carbonées et des gouttelettes d'eau. Ils ont aussi montré qu'à cet angle, les propriétés optiques de diffusion (en intensité) diffèrent le plus d'un type d'amiante à un autre. De plus, à cet angle, on peut éliminer ces autres particules qui ont des propriétés de diffusion lumineuse différentes dans cette zone angulaire.

Suivant d'autres caractéristiques optionnelles du système de détection sur site de particules d'amiante, prises seules ou en combinaison :
- Le premier angle est compris entre 10° et 20°, en particulier 12°.

Ces valeurs permettent de gagner en précision pour distinguer les particules d'amiante par rapport aux autres particules.
- La pompe est apte à avoir un débit d'au moins 2 L/min.
- Le moyen pour libérer les particules d'amiante présentes dans les matériaux est un dispositif pour fragmenter les matériaux de construction.
- Le moyen pour libérer les particules d'amiante est un dispositif pour mettre en suspension des particules d'amiante obtenues à partir de matériaux de construction, par exemple une jarre et un appareil d'agitation pour mettre la jarre en mouvement.
- Le moyen pour libérer les particules d'amiante présentes dans les matériaux est un dispositif pour fragmenter les matériaux de construction, et comprend une jarre destinée à contenir les matériaux de construction, et un appareil d'agitation pour mettre la jarre en mouvement.

La jarre sous agitation permet d'imposer aux matériaux de construction une grande amplitude de mouvement ce qui induit l'attrition ou la fragmentation des matériaux. Ce système de fragmentation sous agitation est particulièrement intéressant car il permet également de mettre ou de remettre en suspension les particules libérées. Ceci optimise le prélèvement des particules par la pompe, ainsi que leur analyse dans le système d'analyse.

De préférence, la jarre peut contenir jusqu'à 850 cm³. Avec une telle capacité de stockage, la jarre présente un faible encombrement, ce qui est avantageux pour un système transportable. Cette capacité de stockage est néanmoins suffisante pour réaliser les tests nécessaires à la détection. De plus, le faible encombrement est associé à un poids réduit. Ainsi, pour mettre en mouvement la jarre, un appareil d'agitation peu puissant suffit, ce qui est associé à une économie d'énergie et à un faible encombrement de l'appareil d'agitation.

De façon avantageuse, la jarre est scellée, donc hermétique, et les connexions entre les éléments du système de détection (jarre, système d'analyse et pompe) sont étanches. Ceci permet à l'opérateur de travailler en toute sécurité.

La jarre peut avoir une contenance d'environ 400 cm³ ou d'environ 120 cm³.

De préférence, l'appareil d'agitation est configuré pour effectuer des mouvements en huit, ce qui permet une grande amplitude de mouvement du matériau au sein de la jarre afin de faciliter l'attrition ou la fragmentation du matériau. Un maximum de particules fines est ainsi libéré en un temps réduit. La durée de mise en mouvement est fonction de la nature du matériau, de ses dimensions et de sa géométrie.

Selon le matériau, des billes de broyage peuvent être ajoutées afin de favoriser la génération des particules fines.
- Le moyen pour libérer les particules d'amiante présentes dans les matériaux est apte à produire des particules de 0,5 à 500 µm.

Cette gamme de tailles est particulièrement adaptée pour la détection de l'amiante par cette technique.
- Le second angle est compris entre 55 et 65°, de préférence égal à environ 60°. A ces valeurs du deuxième angle, les particules de chrysotile et d'amosite sont particulièrement bien discriminées par rapport aux autres particules solides.
- La pompe, le moyen pour libérer les particules d'amiante présentes dans les matériaux, et le système d'analyse des particules solides, sont disposés de sorte que les particules d'amiante libérées peuvent être prélevées pour être transportées jusqu'au système d'analyse des particules solides.
- Un filtre configuré pour retenir des particules d'amiante est placé entre le système d'analyse des particules solides et la pompe.

Le filtre évite que les particules d'amiante ne soient expulsées dans l'air ambiant. Si besoin, ce filtre permet de confirmer la présence d'amiante, à l'aide d'une analyse au microscope électronique à balayage (MEB) ou au microscope électronique en transmission (MET).
- Un filtre à particules est placé en amont du moyen pour libérer les particules d'amiante présentes dans les matériaux.

Ainsi, la présence d'air peut être maintenue au sein du moyen pour libérer les particules d'amiante présentes dans les matériaux, sans y introduire de particules potentiellement présentes dans l'air ambiant, qui pourraient fausser la mesure.
- Le système de détection sur site de particules d'amiante comprend également un ordinateur configuré pour traiter les données issues du système d'analyse des particules solides.

Avantageusement, ledit ordinateur est connecté au système d'analyse des particules solides.
- Le système de détection sur site de particules d'amiante comprend en outre un moyen de piégeage d'au moins une partie du champ lumineux initial. Le moyen de piégeage comprend par exemple un canon optique et un piège à lumière. Le moyen de piégeage est disposé en particulier pour piéger la lumière dont la trajectoire n'a pas été perturbée par les particules. Ainsi, cette lumière non diffusée par les particules est collectée et ne vient pas perturber les mesures.

L'invention a également pour objet un procédé de détection sur site de particules d'amiante dans des matériaux de construction, comprenant les étapes suivantes :
- libération des particules d'amiante présentes dans les matériaux de construction,
- illumination par un champ lumineux initial de particules solides libérées par ledit moyen,
- détection d'un champ lumineux diffusé par lesdites particules solides, ladite étape de détection s'opérant dans une direction formant un angle inférieur à 30° par rapport à la direction du champ lumineux initial, appelé premier angle, et dans une direction formant un angle inférieur compris entre 45° et 90° par rapport à la direction du champ lumineux initial, appelé second angle.

Suivant d'autres caractéristiques optionnelles du procédé de détection, prises seules ou en combinaison :
- Les particules d'amiante libérées sont prélevées, par exemple par pompage, avant d'être illuminées.
- Les particules d'amiante sont libérées par fragmentation, par exemple par mise en agitation des matériaux dans une jarre.
- La libération des particules d'amiante est obtenue par mise en suspension des particules d'amiante.
- Après la fragmentation dans une jarre, celle-ci est connectée à un système d'analyse des particules solides pour procéder à l'étape d'illumination des particules solides.
- Le procédé de détection comprend une étape de piégeage d'au moins une partie d'un champ lumineux initial généré lors de l'étape d'illumination.
- Le procédé de détection comprend une étape dans laquelle on calcule le rapport des intensités des flux détectés aux premier et second angles. On estime ainsi l'importance de la présence de particules d'amiante dans les matériaux de construction.

### Brève description des figures

L'invention sera mieux comprise à la lecture de la description qui va suivre donnée uniquement à titre d'exemple et faite en se référant aux dessins annexés dans lesquels :
[Fig. 1] la figure 1 est une vue schématique d'un système de détection de particules solides selon un état de la technique ;
[Fig. 2] la figure 2 est une vue schématique d'un système de détection sur site de particules d'amiante dans des matériaux de construction selon un mode de réalisation de l'invention.

### Description détaillée

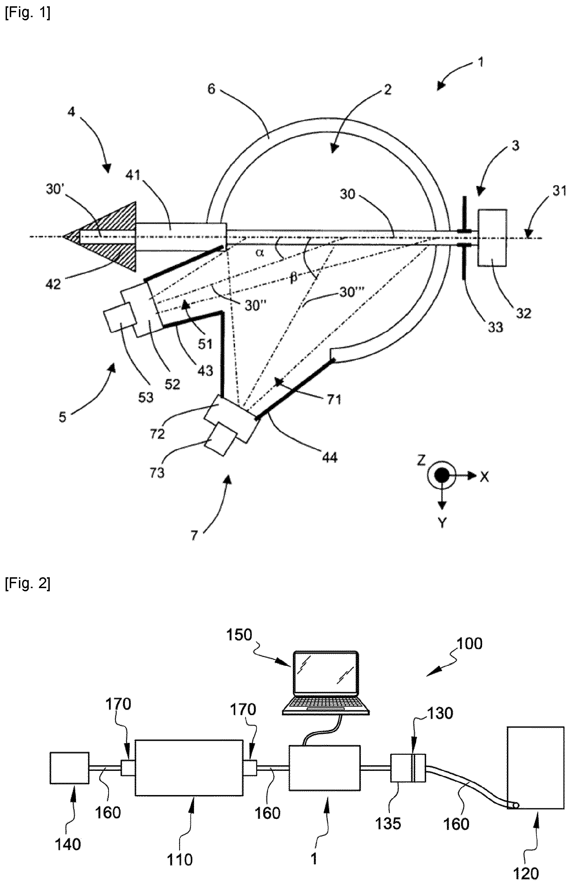

On connaît, par exemple du brevet FR2938649, un procédé et système d'analyse de particules solides dans un milieu. La figure 1 schématise le mode de détection utilisé dans ce système de l'état de la technique. Le système 1 d'analyse des particules solides comprend un dispositif d'illumination 3 pour générer un champ lumineux initial 30 dans une chambre 6 de diffusion de particules solides, présentes dans un milieu 2. Le dispositif d'illumination 3 comprend une source de lumière 32 équipée d'un diaphragme 33.

Le système 1 d'analyse comprend également un moyen principal 5 de détection d'un champ lumineux 30" diffusé par les particules solides. Le moyen principal 5 de la figure 1 comprend une photodiode 52 et un compteur 53. Le moyen principal 5 de détection est orienté dans une direction 51 formant un angle α, dit premier angle, par rapport à la direction 31 du champ lumineux initial 30. Dans ce système d'analyse de l'état de la technique, le premier angle α de diffusion est compris entre 10° et 20°. En ce premier angle α, la lumière diffusée par les particules est insensible à leur indice de réfraction, et donc à leur nature.

Le système 1 d'analyse comprend en outre un moyen complémentaire 7 de détection d'un champ lumineux 30‴ diffusé par les particules solides. Le moyen complémentaire 7 de la figure 1 comprend une photodiode 72 et un compteur 73. Le moyen complémentaire 7 de détection est orienté dans une direction 71 formant un angle β, dit second angle, par rapport à la direction 31 du champ lumineux initial 30. Dans ce système d'analyse de l'état de la technique, le second angle β de diffusion est compris entre 40° et 140°. En ce second angle β, la lumière diffusée dépend fortement de la nature de la particule.

Le système 1 représenté sur la figure 1 comprend également un moyen de piégeage 4 d'une partie 30' du champ lumineux initial 30. Sur la figure, ce moyen de piégeage piège la lumière 30' dont la trajectoire n'a pas été perturbée par les particules. Le moyen de piégeage 4 représenté comprend un canon optique 41 et un piège à lumière 42. Enfin, ce système 1 sur la figure 1 comprend des canons optiques 43, 44, pour ajuster respectivement le champ de vue des détecteurs 5 et 7.

L'utilisation de ce système n'a pas été décrite pour l'étude de l'amiante. Néanmoins, à partir des mesures de courbes de diffusion de la lumière qu'ils ont réalisées dans un autre système, les inventeurs ont déterminé les conditions permettant d'adapter ce système d'analyse de l'état de la technique à la détection spécifique de particules d'amiante.

Les inventeurs ont donc imaginé utiliser ce système d'analyse, qu'ils ont adapté à la détection d'amiante, pour mettre au point un système de détection sur site de particules d'amiante dans des matériaux de construction.

La figure 2 est une vue schématique d'un système 100 de détection sur site de particules d'amiante selon un mode de réalisation de l'invention. Le système 100 de détection comprend un système 1 d'analyse des particules solides similaire à celui schématisé sur la figure 1 et comprenant au moins :
- une source de lumière 32 pour générer un champ lumineux initial 30,
- un moyen principal 5 de détection d'un champ lumineux 30" diffusé par lesdites particules solides, le moyen principal 5 de détection étant orienté dans une direction 51 formant un angle α inférieur à 30° par rapport à la direction 31 du champ lumineux initial 30, appelé premier angle,
- un moyen complémentaire 7 de détection du champ lumineux 30‴ diffusé par lesdites particules solides, le moyen complémentaire 7 de détection étant orienté dans une direction 71 formant un angle β entre 45° et 90° par rapport à la direction 31 du champ lumineux initial 30, appelé second angle.

Le système 100 de détection comprend en outre un moyen 110 pour libérer les particules d'amiante présentes dans les matériaux de construction, et une pompe 120 pour prélever des particules solides libérées par le moyen 110 pour libérer les particules d'amiante.

Dans le mode de réalisation représenté sur la figure 2, le moyen 110 pour libérer les particules d'amiante, qui est un dispositif pour fragmenter les matériaux de construction. Il comprend une jarre dans laquelle sont placés les matériaux de construction et un appareil d'agitation pour mettre la jarre en mouvement. La jarre permet également de mettre ou remettre en suspension des particules d'amiante obtenues à partir de matériaux de construction. La jarre est généralement en inox. En général, la jarre peut contenir jusqu'à 850 cm³. La jarre peut également avoir une contenance d'environ 400 cm³ ou 120 cm³. L'appareil d'agitation peut par exemple effectuer des mouvements en huit.

Un moyen 110 capable de produire des particules de 0,5 à 500 µm est particulièrement adapté pour la détection d'amiante par cette technique.

Il est avantageux que le moyen de détection complémentaire 7 soit orienté dans une direction 71 formant un angle β entre 55 et 65°, de préférence d'environ 60°, afin de bien discriminer les particules de chrysotile et d'amosite par rapport aux autres particules solides.

Dans le mode de réalisation représenté sur la figure 2, la pompe 120, le moyen 110 pour libérer les particules d'amiante présentes dans les matériaux, et le système 1 d'analyse des particules solides, sont disposés de sorte que les particules d'amiante libérées par le moyen 110 peuvent être prélevées pour être transportées jusqu'au système 1 d'analyse des particules solides. Un filtre 130 configuré pour retenir des particules d'amiante est placé entre le système 1 d'analyse des particules solides et la pompe 120. Le filtre 130 est porté par une cassette porte-filtre 135. De plus, un filtre à particules 140 est placé en amont du moyen 110 pour libérer les particules d'amiante présentes dans les matériaux. Le système 100 de détection comprend également un ordinateur 150 configuré pour traiter les données issues du système 1 d'analyse des particules solides. L'ordinateur 150 est connecté au système 1 d'analyse. Le système 1 d'analyse comprend en outre un moyen de piégeage 4 d'au moins une partie du champ lumineux initial 30. Le moyen de piégeage 4, qui comprend par exemple un canon optique 41 et un piège à lumière 42, est disposé pour piéger la lumière 30' dont la trajectoire n'a pas été perturbée dans la chambre de diffusion 6 par les particules.

Les éléments du système 100 de détection (jarre 110, système d'analyse 1 et pompe 120) sont reliés entre eux par des connexions 160 étanches. La jarre 110 est reliée aux autres éléments du système 100 de détection en utilisant des raccords rapides 170.

A l'aide du système de détection 100 de la figure 2, on détecte sur site les particules d'amiante dans des matériaux de construction en mettant en œuvre les étapes suivantes :
- libération des particules d'amiante présentes dans les matériaux de construction,
- illumination par un champ lumineux initial 30 des particules solides libérées,
- détection d'un champ lumineux 30" diffusé par lesdites particules solides, ladite étape de détection s'opérant dans une direction 51 formant un angle α inférieur à 30° par rapport à la direction du champ lumineux initial 30, appelé premier angle, et dans une direction 71 formant un angle β compris entre 45° et 90° par rapport à la direction 31 du champ lumineux initial 30, appelé second angle.

Les particules d'amiante libérées peuvent être prélevées, par exemple par pompage, avant d'être illuminées. Le procédé de détection peut comprendre une étape de piégeage d'au moins une partie 30' du champ lumineux initial 30. Le procédé de détection peut comprendre une étape dans laquelle on calcule le rapport des intensités des flux détectés aux premier angle α et second angle β.

## Revendications

1. Système (100) de détection sur site de particules d'amiante dans des matériaux de construction comprenant :
- un moyen (110) pour libérer les particules d'amiante présentes dans les matériaux,
- une pompe (120) configurée pour prélever des particules solides libérées par ledit moyen,
- un système (1) d'analyse des particules solides comprenant :
une source de lumière (32) configurée pour générer un champ lumineux initial (30),
un moyen principal (5) de détection d'un champ lumineux (30") diffusé par lesdites particules solides, le moyen principal (5) de détection étant orienté dans une direction (51) formant un angle α inférieur à 30° par rapport à la direction (31) du champ lumineux initial (30), appelé premier angle,
un moyen complémentaire (7) de détection du champ lumineux (30‴) diffusé par lesdites particules solides, le moyen complémentaire (7) de détection étant orienté dans une direction (71) formant un angle β entre 45° et 90° par rapport à la direction (31) du champ lumineux initial (30), appelé second angle.

2. Système (100) de détection sur site de particules d'amiante selon la revendication précédente, dans lequel le moyen (110) pour libérer les particules d'amiante présentes dans les matériaux est un dispositif pour fragmenter les matériaux de construction.

3. Système (100) de détection sur site de particules d'amiante selon l'une quelconque des revendications précédentes, dans lequel le moyen (110) pour libérer les particules d'amiante présentes dans les matériaux est un dispositif pour mettre en suspension des particules d'amiante obtenues à partir de matériaux de construction.

4. Système (100) de détection sur site de particules d'amiante selon la revendication 2, dans lequel le dispositif pour fragmenter les matériaux de construction comprend une jarre destinée à contenir les matériaux de construction, et un appareil d'agitation pour mettre la jarre en mouvement.

5. Système (100) de détection sur site de particules d'amiante selon l'une quelconque des revendications précédentes, dans lequel le moyen (110) pour libérer les particules d'amiante présentes dans les matériaux est configuré pour produire des particules de 0,5 à 500 µm.

6. Système (100) de détection sur site de particules d'amiante selon l'une quelconque des revendications précédentes, dans lequel le second angle β est compris entre 55 et 65°, de préférence égal à environ 60°.

7. Système (100) de détection sur site de particules d'amiante selon l'une quelconque des revendications précédentes, dans lequel la pompe (120), le moyen (110) pour libérer les particules d'amiante présentes dans les matériaux, et le système (1) d'analyse des particules solides, sont disposés de sorte que les particules d'amiante libérées peuvent être prélevées pour être transportées jusqu'au système (1) d'analyse des particules solides.

8. Système (100) de détection sur site de particules d'amiante selon l'une quelconque des revendications précédentes, dans lequel un filtre (130) configuré pour retenir des particules d'amiante est placé entre le système (1) d'analyse des particules solides et la pompe (120).

9. Système (100) de détection sur site de particules d'amiante selon l'une quelconque des revendications précédentes, dans lequel un filtre à particules (140) est placé en amont du moyen (110) pour libérer les particules d'amiante présentes dans les matériaux.

10. Système (100) de détection sur site de particules d'amiante selon l'une quelconque des revendications précédentes, comprenant également un ordinateur (150) configuré pour traiter les données issues du système (1) d'analyse des particules solides.

11. Système (100) de détection sur site de particules d'amiante selon l'une quelconque des revendications précédentes, comprenant en outre un moyen de piégeage (4) d'au moins une partie du champ lumineux initial (30).

12. Procédé de détection sur site de particules d'amiante dans des matériaux de construction, comprenant les étapes suivantes :
- libération des particules d'amiante présentes dans les matériaux de construction,
- illumination par un champ lumineux initial (30) de particules solides libérées,
- détection d'un champ lumineux (30") diffusé par lesdites particules solides, ladite étape de détection s'opérant dans une direction (51) formant un angle α inférieur à 30° par rapport à la direction du champ lumineux initial (30), appelé premier angle, et dans une direction (71) formant un angle β compris entre 45° et 90° par rapport à la direction (31) du champ lumineux initial (30), appelé second angle.

13. Procédé de détection selon la revendication précédente, dans lequel la libération des particules d'amiante est obtenue par fragmentation des matériaux de construction.

14. Procédé de détection selon la revendication 12, dans lequel la libération des particules d'amiante est obtenue par mise en suspension des particules d'amiante.

## Patentansprüche

1. System (100) zur Vor-Ort-Detektion von Asbestpartikeln in Baumaterialien, aufweisend:
- ein Mittel (110) zum Freisetzen von Asbestpartikeln, die in den Materialien vorhanden sind,
- eine Pumpe (120), die konfiguriert ist, die von dem Mittel freigesetzten Feststoffpartikel aufzunehmen,
- ein System (1) zur Analyse der Feststoffpartikel, aufweisend:
eine Lichtquelle (32), die konfiguriert ist, ein anfängliches Lichtfeld (30) zu erzeugen,
ein Hauptmittel (5) zur Detektion eines Lichtfelds (30"), das von den Feststoffpartikeln gestreut wird, wobei das Hauptmittel (5) zur Detektion in eine Richtung (51) gerichtet ist, die einen Winkel α von weniger als 30° in Bezug auf die Richtung (31) des anfänglichen Lichtfelds (30) bildet, erster Winkel genannt,
ein komplementäres Mittel (7) zur Detektion des von den Feststoffpartikeln gestreuten Lichtfeldes (30‴), wobei das komplementäre Detektionsmittel (7) in einer Richtung (71) ausgerichtet ist, die einen Winkel β zwischen 45° und 90° in Bezug auf die Richtung (31) des ursprünglichen Lichtfeldes (30) bildet, zweiter Winkel genannt.

2. System (100) zur Vor-Ort-Detektion von Asbestpartikeln nach dem vorhergehenden Anspruch, wobei das Mittel (110) zum Freisetzen der in den Materialien vorhandenen Asbestpartikel eine Vorrichtung zum Zerkleinern der Baumaterialien ist.

3. System (100) zur Vor-Ort-Detektion von Asbestpartikeln nach einem der vorhergehenden Ansprüche, wobei das Mittel (110) zur Freisetzung der in den Materialien vorhandenen Asbestpartikel eine Vorrichtung zum Suspendieren von Asbestpartikeln ist, die aus Baumaterialien gewonnen wurden.

4. System (100) zur Vor-Ort-Detektion von Asbestpartikeln nach Anspruch 2, wobei die Vorrichtung zum Zerkleinern der Baustoffe ein Gefäß zur Aufnahme der Baustoffe und eine Rührvorrichtung aufweist, um das Gefäß in Bewegung zu versetzen.

5. System (100) zur Vor-Ort-Detektion von Asbestpartikeln nach einem der vorhergehenden Ansprüche, wobei die Mittel (110) zum Freisetzen von Asbestpartikeln, die in den Materialien vorhanden sind, konfiguriert ist, Partikel mit einer Größe von 0,5 bis 500 µm zu erzeugen.

6. System (100) zur Vor-Ort-Detektion von Asbestpartikeln nach einem der vorhergehenden Ansprüche, wobei der zweite Winkel (3) zwischen 55 und 65°, vorzugsweise etwa 60°, beträgt.

7. System (100) zur Vor-Ort-Detektion von Asbestpartikeln nach einem der vorhergehenden Ansprüche, wobei die Pumpe (120), die Mittel (110) zum Freisetzen der in den Materialien vorhandenen Asbestpartikel und das System (1) zur Analyse der Feststoffpartikel (1) so angeordnet sind, dass die freigesetzten Asbestpartikel zum Transport zu dem System (1) zur Analyse der Feststoffpartikel entnommen werden können.

8. System (100) zum Vor-Ort- Detektion von Asbestpartikeln nach einem der vorhergehenden Ansprüche, wobei ein Filter (130), der konfiguriert ist, Asbestpartikel zurückzuhalten, zwischen dem System (1) zur Analyse der Feststoffpartikel und der Pumpe (120) angeordnet ist.

9. System (100) zur Vor-Ort- Detektion von Asbestpartikeln nach einem der vorhergehenden Ansprüche, wobei ein Partikelfilter (140) stromaufwärts der Mittel (110) zur Freisetzung von Asbestpartikeln in den Materialien angeordnet ist.

10. System (100) zur Vor-Ort-Detektion von Asbestpartikeln nach einem der vorhergehenden Ansprüche, das auch einen Computer (150) aufweist, der konfiguriert ist, die vom System (1) zur Analyse von Feststoffpartikeln stammenden Daten zu verarbeiten.

11. System (100) zur Vor-Ort-Detektion von Asbestpartikeln nach einem der vorhergehenden Ansprüche, ferner aufweisend ein Mittel zum Einfangen (4) von mindestens einem Teil des ursprünglichen Lichtfeldes (30).

12. Verfahren zur Vor-Ort-Detektion von Asbestpartikeln in Baumaterialien, umfassend die folgenden Schritte:
- Freisetzen der in den Baumaterialien vorhandenen Asbestpartikel,
- Beleuchten der freigesetzten Feststoffpartikel mit einem anfänglichen Lichtfeld (30),
- Detektieren eines Lichtfeldes (30"), das von den Feststoffpartikeln gestreut wird, wobei der Schritt des Detektierens in einer Richtung (51) erfolgt, die einen Winkel α von weniger als 30° mit der Richtung des anfänglichen Lichtfeldes (30) bildet, der als erster Winkel bezeichnet wird, und in einer Richtung (71), die einen Winkel β zwischen 45° und 90° mit der Richtung (31) des anfänglichen Lichtfeldes (30) bildet, der als zweiter Winkel bezeichnet wird.

13. Detektionsverfahren nach dem vorhergehenden Anspruch, bei dem das Freisetzen der Asbestpartikel durch Zerkleinerung der Baustoffe erreicht wird.

14. Detektionsverfahren nach Anspruch 12, bei dem das Freisetzen der Asbestpartikel durch Aufwirbeln der Asbestpartikel erreicht wird.

## Claims

1. System (100) for the on-site detection of asbestos particles in building materials comprising:
- a means (110) for releasing asbestos particles present in materials,
- a pump (120) configured to take solid particles released by said means,
- a solid particle analysis system (1) comprising:
a light source (32) configured to generate an initial light field (30), a main means (5) for detecting a light field (30") scattered by said solid particles, the main means (5) for detecting being oriented in a direction (51) forming an angle α less than 30° with respect to the direction (31) of the initial light field (30), called the first angle,
an additional means (7) for detecting the light field (30‴) scattered by said solid particles, the additional means (7) for detecting being oriented in a direction (71) forming an angle β between 45° and 90° with respect to the direction (31) of the initial light field (30), called the second angle.

2. System (100) for on-site detection of asbestos particles according to the preceding claim, wherein the means (110) for releasing asbestos particles present in materials is a device for breaking up building materials.

3. System (100) for on-site detection of asbestos particles according to any one of the preceding claims, wherein the means (110) for releasing asbestos particles from materials is a device for suspending asbestos particles obtained from building materials.

4. System (100) for on-site detection of asbestos particles according to claim 2, wherein the device for breaking up the building materials comprises a jar for containing the building materials, and a stirring apparatus for setting the jar in motion.

5. System (100) for on-site detection of asbestos particles according to any one of the preceding claims, wherein the means (110) for releasing asbestos particles present in materials is configured to produce particles from 0.5 to 500 µm.

6. System (100) for on-site detection of asbestos particles according to any one of the preceding claims, wherein the second angle β is between 55 and 65°, preferably about 60°.

7. System (100) for on-site detection of asbestos particles according to any one of the preceding claims, wherein the pump (120), the means (110) for releasing asbestos particles present in materials, and the system (1) for analyzing solid particles, are arranged so that the released asbestos particles can be sampled for transport to the solid particle analysis system (1).

8. System (100) for on-site detection of asbestos particles according to any one of the preceding claims, wherein a filter (130) configured to retain asbestos particles is placed between the solid particle analysis system (1) and the pump (120).

9. System (100) for on-site detection of asbestos particles according to any one of the preceding claims, wherein a particulate filter (140) is placed upstream of the means (110) for releasing asbestos particles present in the materials.

10. System (100) for on-site detection of asbestos particles according to any one of the preceding claims, also comprising a computer (150) configured to process data from the system (1) for analyzing solid particles.

11. System (100) for on-site detection of asbestos particles according to any one of the preceding claims, further comprising a means of trapping (4) at least part of the initial light field (30).

12. A method for the on-site detection of asbestos particles in building materials, comprising the following steps:
- release of asbestos particles present in construction materials,
- illumination by an initial light field (30) of released solid particles,
- detection of a light field (30") scattered by said solid particles, said detection step operating in a direction (51) forming an angle α of less than 30° with respect to the direction of the initial light field (30), called the first angle, and in a direction (71) forming an angle β between 45° and 90° with respect to the direction (31) of the initial light field (30), called the second angle.

13. Method of detection according to the preceding claim, wherein the release of asbestos particles is obtained by breaking up the building materials.

14. Method of detection according to claim 12, wherein the release of asbestos particles is obtained by suspending the asbestos particles.
